# EUROPÄISCHE PATENTSCHRIFT

(11) **EP 0 920 618 B1**
(45) Veröffentlichungstag und Bekanntmachung des Hinweises auf die Patenterteilung: **14.12.2005**
(21) Anmeldenummer: 97941958.7
(22) Anmeldetag: 22.08.1997
(51) Int. Cl.: G01N 27/327, C12G 1/00

(54) **NEUARTIGE DÜNNSCHICHTEN FÜR DIE MIKROSYSTEMTECHNIK UND MIKROSTRUKTURIERUNG SOWIE IHRE VERWENDUNG**
NEW KIND OF THIN FILMS FOR MICROSYSTEM TECHNOLOGY AND MICROSTRUCTURING AND THEIR USE
NOUVEAUX TYPES DE FILMS MINCES POUR LA TECHNIQUE DES MICROSYSTEMES ET LA MICROSTRUCTURATION, ET LEUR UTILISATION

(30) Priorität: 23.08.1996 DE 19634120; 15.02.1997 DE 19705909
(43) Veröffentlichungstag der Anmeldung: 09.06.1999
(73) Patentinhaber: Clondiag Chip Technologies GmbH, 07743 Jena (DE)
(72) Erfinder: ERMANTRAUT, Eugen, D-07745 Jena (DE); KÖHLER, Johann, Michael, D-07751 Golmsdorf (DE); SCHULZ, Torsten, D-07743 Jena (DE); WOHLFART, Klaus, D-07751 Laasan (DE); WÖLFL, Stefan, D-07745 Jena (DE)
(74) Vertreter: Maiwald Patentanwalts GmbH
(86) Internationale Anmeldenummer: PCT/EP1997/004582
(87) Internationale Veröffentlichungsnummer: WO 1998/008086

(56) Entgegenhaltungen:
- EP-A- 0 110 483
- EP-A- 0 116 361
- EP-A- 0 246 602
- DD-A- 298 268
- US-A- 5 846 814
- DATABASE WPI Section Ch, Week 8907 Derwent Publications Ltd., London, GB; Class B04, AN 89-049890 XP002051423 & JP 01 005 489 A (FUJI PHOTO FILM CO LTD) , 10.Januar 1989
- MCCONNELL H.M. ET AL: 'SUPPORTED PLANAR MEMBRANES IN STUDIES OF CELL-CELL RECOGNITION IN THE IMMUNE SYSTEM' BIOCHIMICA ET BIOPHYSICA ACTA Bd. 864, 1986, Seiten 95 - 106

## Beschreibung

Neuartige Dünnschichten für die Mikrosystemtechnik und Mikrostrukturierung sowie ihre Verwendung

Die Erfindung betrifft eine Anordnung gemäß Anspruch 1 und ein Verfahren gemäß Anspruch 23.

Bislang nach dem Stand der Technik in der Mikrosystemtechnik und Mikrostrukturierung eingesetzte Dünnschichten, bspw. zur Erzeugung von Membranen, Kontakt- und Leitbahnschichtsystemen, basieren auf dem Einsatz anorganischer Schichten, wie häufig verwendeter Schichten aus SiO₂, Si₃N₄, Al₂O₃, und Metallschichten bzw. Metallschichtsystemen. Zur Erzeugung der gewünschten Strukturen kommen dabei gesundheitsschädigende und giftige Chemikalien, wie z.B. starke Säuren, Laugen und Oxidationsmittel zum Einsatz oder es sind äußerst kostenaufwendige Prozesse, wie reaktives Ionen- bzw. Plasmaätzen erforderlich (S. Büttgenbach, Mikromechanik, B. G. Teubner Stuttgart, 1994). Die einzigen organischen Schichten, die innerhalb dieser Strukturerzeugungsprozesse Verwendung finden, sind Fotoresiste, die nach der Übertragung der gewünschten Struktur in die zu strukturierende Schicht in der Regel wieder entfernt werden.

Aus der Ztschr. Biochimica et Biophysica Acta 864 (1986) 95-106 (H. M. McConnell et al) ist es bekannt, ebene Membranen im Tauchverfahren herzustellen, die zum Studium von Zell-Zell-Erkennungen in Immunsystemen verwendet werden. Wegen der nicht immer regelmäßigen Membrandicken von mindestens einigen Mikrometern sind die Moleküle in der Mehrzahl der Fälle nicht zugänglich, so dass die Membranen keinesfalls zu Zwecken der Mikrostrukturierung geeignet sind.

Dokument FR-A-2245009 offenbart eine Anordnung, umfassend ein Trägersubstrat auf dem eine enzymatisch abbaubare Biopolymer-Dünnschicht (eine 10-500 µm dicke Photogelantineschicht), die einen photoempfindlichen enzymatischen Inhibitor enthält, aufgebracht ist. Das Dokument ist auschliesslich im Bereich der Photographietechniken angesiedelt.

Dokument US-A-5154808 offenbart eine Anordnung, umfassend ein Trägersubstrat auf dem eine Dünnschicht (0.5-10 µm) aufgebracht ist, die lichtaktive Kopplungsmoleküle (precursor) enthält, mit dem bioaktive Moleküle indirekt auf dem Trägersubstrat immobilisiert werden können. Ein Bindemittel (Gelatine) fixiert die Kopplungsmoleküle an das Trägersubstrat.

Der Erfindung liegt die Aufgabe zugrunde, Dünnschichten anzugeben, die sich problemloser und kostengünstiger als bislang übliche Schichten herstellen lassen und die Verwendung der vorhandenen Technologien der Mikrostrukturierung zulassen, und die sich in besonders vorteilhafter Weise für den Aufbau von Substanzbibliotheken eignen.

Die Aufgabe wird durch die kennzeichnenden Merkmale des ersten Patentanspruchs gelöst. Vorteilhafte Ausgestaltungen sind durch die nachgeordneten Ansprüche erfaßt.

Es wurde gefunden, daß im Bereich der Mikrostrukturierung bislang zum Einsatz gelangende, strukturierbare Maskierungsschichten, wie Fotoresistschichten, allein durch ihre Anwesenheit lokal die Eigenschaften von Biopolymerfilmen, z.B. Gelatine, Agarose, Dextrose und Lipide, beeinflussen können. Überraschend wurde weiterhin gefunden, daß sich solche Biopolymerfilme auch in dem für die Dünnschichttechnik relevanten Dickenbereich von 30 nm - 3 µm in sehr hoher Qualität herstellen lassen. Außerdem wurde gefunden, daß bestimmte Farbstoffe oder photoaktivierbare Gruppen nach einer erfolgten photochemischen Aktivierung eine Vernetzung der Biopolymerschicht derart bewirken, daß sie enzymatisch deutlich langsamer abbaubar sind, als unbelichtete Schichtbereiche.

Die Schichtdicken lassen sich durch das an sich bekannte spin-coating aus Lösungen mit einem Feststoffgehalt von 1-30%, im Bereich von einigen 10 nm reproduzieren. Die so aufgetragenen Schichten sind selbst in diesem extremen Dünnschichtbereich unerwarteter Weise homogen und defektfrei. Ebenso halten sie im weiteren Prozeß der Mikrostrukturierung häufig erforderlichen Temperschritten bis 250 °C ohne Degradationserscheinungen problemlos Stand.

Der Hauptvorteil dieser Schichten aus Biopolymeren besteht jedoch in ihrer enzymatischen Abbaubarkeit, was eine hohe Spezifität des Abbaus zur Folge hat, der in der Regel unter moderaten Bedingungen, bei Raumtemperatur, in Lösungen mit pH-Werten vorzugsweise zwischen 4 - 9, erfolgt. Grundsätzlich bieten Biopolymere den Vorteil, daß sie definierte Funktionen zur kovalenten oder auch nichtkovalenten Ankopplung von weiteren Molekülen und Schichten bieten, so kann bspw. an eine Gelatineschicht über Amino-, Carboxy-, Thiofunktionen als auch über Wasserstoffbrückenbindungen gekoppelt werden. Weiterhin ist es problemlos möglich, Biopolymere zu vernetzen (z.B. mittels Glutardialdehyds bei Vorliegen von freien Keto-, Amino-, Hydroxygruppen) und damit die Eigenschaften der erzeugten Schicht entsprechend zu verändern, so wird z.B. aus wasserlöslicher Gelatine nach dem Quervernetzen ein nicht wasserlösliches Material, in dem Moleküle eingeschlossen oder aber an das diese kovalent und nichtkovalent gebunden werden können. Je nach Ausbildung können die vorgeschlagenen Dünnschichten im vorgesehenen Anwendungsgebiet zu vielfältigen Zwecken verwendet werden. Eine nähere Illustration dieser Anwendungsmöglichkeiten wird in folgenden Ausführungsbeispielen aufgezeigt.

In einem Referenzbeispiel wird die Eigenschaft von Enzymen als hochspezifische Katalysatoren angewandt, um eine freitragende Novolacstruktur unter Einsatz einer aus Gelatine bestehenden biopolymeren Dünnschicht zu erzeugen. Dazu wird auf einen gereinigten Siliziumwafer eine ca. 200 nm dicke Gelatineschicht durch Aufschleudern (spin-coating) aufgebracht. Im Beispiel wird diese Schicht durch in Wasser gelöste Gelatine (10% v/v), die mit 5% Glutardialdehyd versetzt wird gebildet. Diese Schicht ist nicht wasserlöslich und gegen übliche Fotolackentwickler resistent. Auf diese Schicht wird ebenfalls mittels spin-coating eine handelsübliche Fotoumkehrresist aufgebracht. Die Fotoresistschicht wird nach Vorschrift behandelt, entsprechend ihrer später gewünschten Struktur maskiert, belichtet und strukturiert. Der gesamte Schichtverbund in einen Behälter mit einem enzymatisches Bad eingebracht. Im Falle der Verwendung von Gelatine für die Biopolymerschicht besteht das enzymatische Bad bevorzugt aus einem Protease K - Puffer, der im wesentlichen durch 10% SDS, 10 mM NaCl, 10 mM EDTA und Tris-HCl gebildet wird und dem 10 mg/ml Protease K zugegeben sind. Der pH-Wert dieses Bades ist auf 8,5 eingestellt. Unter Einsatz eines solchen enzymatischen Bades erfolgt der vollständige Abbau der ca. 200 nm dicken Gelatineschicht bei Raumtemperatur innerhalb von ca. 8 h. In diesem Beispiel fand die Biopolymerschicht Anwendung als Opferschicht zur Generierung einer freitragenden Novolacstruktur.

In einem zweiten Ausführungsbeispiel, bei dem wieder von einem nach dem ersten Beispiel ausgebildeten Schichtverbund aus Gelatine und einem Fotoresist ausgegangen wird, wird die Eigenschaft von Enzymen als hochspezifische Katalysatoren, die in ihrer Funktion durch geeignete Inhibitoren bzw. Kompetitoren gehemmt werden, angewandt. So bindet Diazonaphtochinon, die in AZ-Fotoresists übliche fotosensitive Komponente, an OH-Gruppen der Gelatine und hindert somit eine in einer Pufferlösung eingesetzte Protease am Abbau. Beim Belichten wandelt sich das Diazonaphtochinon in eine Carboxylsäure um, die als Salz aus der Gelatine gelöst wird, so kann an solchen Stellen der Abbau ungehindert erfolgen. Der Abbau erfolgt somit in Analogie zu bisherigen selektiven Ätztechniken "anisotrop". An Stellen, an denen der Inhibitor nach wie vor vorhanden ist, ist die Abbaugeschwindigkeit deutlich herabgesetzt. Damit ist der Einsatz von Biopolymeren und den jeweils passenden Abbau- bzw. Modifikationsenzymen sowohl als mikrosystemtechnische Komponente als auch als Maskenmaterial gegeben.

In einem dritten Ausführungsbeispiel besteht die Möglichkeit, die Biopolymerdünnschichten mit einem lichtempfindlichen Zusatz (z.B. Diazonaphtochinon) zu versetzen und damit eine Schicht zu schaffen, die selbst als Fotoresist einsetzbar ist. Voraussetzung hierfür ist, daß der lichtempfindliche Zusatz entweder selbst als Inhibitor für das abbauende Enzym wirkt oder an einen solchen Inhibitor gekoppelt ist. Durch diese geschaffene Ausführungsmöglichkeit lassen sich enzymatisch entwickelbare Fotoresists herstellen.

In einem vierten Ausführungsbeispiel soll die Herstellung einer ca. 100 nm dicken Lipidschicht beschrieben werden. Dazu wird eine Lösung bestehend aus Phosphotidylethanolamin in Chloroform (0,1 g/ml) bei 5000 U/min in 30 s auf ein geeignetes Substrat aufgeschleudert.

In einem fünften Ausführungsbeispiel besteht die Möglichkeit, eine Dünnschicht aus Gelatine versetzt mit einem biogenen Inhibitor der Proteasefunktion wie z.B. TFPI (tissue factor pathway inhibitor) 1 : 1000 Massenanteilen gemischt, mit einer Dünnschicht aus reiner Gelatine zu überschichten. Damit ist im Falle einer nachträglichen enzymatischen Behandlung ein Ätzstopp geschaffen. In analoger Weise kann unter Auswahl eines für das nachträglich eingesetzte Enzym inhibierend wirkender Substanz ein Abbaustopp in Schichten bestehend aus Agarose, Dextrose und Lipiden eingebracht werden.

Während die bisher beschriebenen Ausführungsbeispiele die Ausbildung von Dünnschichten betreffen, die vergleichbar wie ein nach dem Stand der Technik bekannter Positivfotoresist anwendbar sind, soll im folgenden sechsten Ausführungsbeispiel eine Dünnschicht gemäß der Erfindung beschrieben werden, die einem Negativresist vergleichbar ist.

Für die photoaktive Komponente mit einer spektralen Empfindlichkeit im sichtbaren oder ultravioletten Spektraibereich, z.B. ein Diazidostilben mit einem Empfindlichkeitsmaximum bei ca. 345 nm, welches in Wasser gelöst wird. Als besonders vorteilhaft hat sich dabei ein Natriumsalz der 4,4-Diazidostilben-2,2-Sulfonsäure erwiesen, welches in Wasser mit einem Mischungsverhältnis von 1 :50 bis 1 : 100 gelöst wird. Die wässrige Diazidostilbenlösung wird, je nach gewünschtem Vernetzungsgrad in der herzustellenden Biopolymerschicht, in einem Mengenverhältnis von 10 : 1 bis 100 : 1 mit einer festen Gelatine, z.B. der Qualität Bloom 60 aus Schweinehaut, versetzt. Dabei werden im Beispiel ca. 40 mg Gelatine pro Milliliter Diazidostilbenlösung eingesetzt. Nach Auflösung der Gelatine wird die Lösung mit einem Mikrofilter auf 200 nm filtriert. Die so erhaltene Lösung wird auf ein Substrat - bspw. bestehend aus Metall, Polymer, Silizium, beschichtetes Silizium oder Glas -, wie in der Mikrolithografie üblich, aufgeschleudert. Im Beispiel wird so auf einem Siliziumsubstrat eine ebene homogene Gelatineschicht mit einer Dicke von ca. 100 nm gebildet. Diese Dünnschicht wird mit einer, der später gewünschten Struktur angepaßten Maske versehen und einer UV-Belichtung bei einer Wellenlänge von 360 nm über ca. 400 s unterworfen. Dabei erfolgt eine photochemische Spaltung der Azidogruppen des Diazidostilbens zu einem Bisnitrenradikal unter Abspaltung von Stickstoff, was zu einer Vernetzung der Aminosäureketten der Gelatine in den belichteten Bereichen führt. Die Entwicklung der belichteten Gelatineschicht erfolgt in Wasser, was eine grobe Ablösung der unbelichteten Bereiche der Gelatineschicht bereits nach 1 - 2 min bewirkt. Daran schließt sich eine Nachentwicklung mit einer Protease-Pufferlösung mit einer Konzentration von 0,1 mg/ml Pufferlösung, im Beispiel bestehend aus 10% Natriumdodecylsulfat, 10 mM NaCl, 10 mM EDTA, Tris-HCl, bei einem schwach alkalischen pH-Wert um ca. 8,5, an. Die dabei im Beispiel erzielbaren Abbauraten liegen bei 20 - 30 nm/min. Auf die beschriebene Weise sind Strukturgrößen bis hinuter zu 1 µm erzeugbar.

Durch die Art der im Rahmen dieser Erfindung erzeugten Quervemetzung der belichteten Bereiche sind sogar noch stabilere Strukturen herstellbar, als der nach den Ausführungsbeispielen eins bis fünf geschaffenen Positivstrukturen.

Der Vorteil von Biopolymeren, daß sie definierte Funktionen zur kovalenten oder auch nichtkovalenten Ankopplung von weiteren Molekülen bieten, so kann bspw. an eine Gelatineschicht über Amino-, Carboxy-, Hydroxi- oder Thiofunktionen als auch über Wasserstoffbrückenbindungen gekoppelt werden, kann in einer speziellen Verwendung geeignet strukturierter, nach obigen Maßgaben gefertigter Dünnschichtstrukturen besonders vorteilhaft ausgenutzt werden, die im folgenden beschrieben wird.

Ausgehend von einer Gelatineschicht, die nach obigen Maßgaben hergestellt ist, Auflegung einer z.B. schachbrettartig gestalteten Maske und Durchführung einer analog zu oben beschriebenen Belichtung und Entwicklung, lassen sich auf einem Trägersubstrat regelmäßige Anordnungen von Quadraten fertigen. Sollen den Quadraten Kantenlängen von bspw. 16 µm gegeben sein, besitzen sie nach vollständiger Durchführung obigen Entwicklungsprozesses eine Strukturhöhe von 18 nm. Solcherart gefertigte Mikrostrukturen (Pads) mit vordefinierten Bindungsstellen aus Gelatine bzw. verwandten Kollagenen eignen sich hervorragend für den Aufbau von Screeningtests und Substanzbibliotheken, die zum schnellen Auffinden von wechselwirkenden Partnern auf molokularer Ebene dienen, für die Biotechnologie, Mölekularbiologie, Pharmazie und Medizin. An den mikrostrukturierten Gelatinepads lassen sich leicht über eine Peptidbindung Proteine (z.B. Antikörper) oder entsprechend modifizierte Oligonukleotide ankoppeln, wodurch z.B. Testassays aufbaubar sind.

Mit den strukturierten Gelatinepads sind lokale Reaktionsräume definiert. Aufgrund der chemischen Diversität der funktionellen Gruppen des Kollagenpolymers ist die Anbindung diverser Moleküle nach spezifischer Aktivierung möglich. Das heißt, die gleiche Matrix kann zur Kopplung von Molekülen mit mannigfaltigen funktionellen Gruppen genutzt werden, es entfällt die aufwendige Modifikation der Moleküle zur Immobilisierung.

## Patentansprüche

1. Anordnung, umfassend ein Trägersubstrat, auf dem eine Dünnschicht aus einem enzymatisch abbaubaren Biopolymer angebracht ist, die eine Schichtdicke von 30 nm bis 3 µm aufweist und partiell, ganzflächig oder volumenmäßig durchsetzt mit einem Inhibitor für den enzymatischen Abbau versehen ist, wobei an das Biopolymer Proteine und/oder Oligonukleotide angekoppelt sind.

2. Anordnung nach Anspruch 1,
**dadurch gekennzeichnet, dass** die Dünnschicht aus einem enzymatisch abbaubaren Biopolymer, ausgewählt aus der Gruppe bestehend aus Gelatine, Agarose, Dextrose und/oder einem Lipid, gebildet ist.

3. Anordnung nach Anspruch 1 oder 2,
**dadurch gekennzeichnet, dass** die enzymatisch abbaubare Dünnschicht mit lichtempfindlichen Zusätzen als Inhibitoren für den enzymatischen Abbau versehen ist.

4. Anordnung nach einem der Ansprüche 1 bis 3,
**dadurch gekennzeichnet, dass** der Inhibitor eine Verbindung ist, die die Funktion eines das Biopolymer abbauenden Enzyms hemmt.

5. Anordnung nach Anspruch 3 oder 4,
**dadurch gekennzeichnet, dass** der lichtempfindliche Zusatz Diazonaphtochinon ist.

6. Anordnung nach einem der vorhergehenden Ansprüche,
**dadurch gekennzeichnet, dass** die Dünnschicht durch Zusatz thermisch aktivierbarer Reagenzien vernetz, insbesondere quervernetzt ist.

7. Anordnung nach Anspruch 6,
**dadurch gekennzeichnet, dass** das thermisch aktivierbare Reagenz Glutardialdehyd oder Formaldehyd ist.

8. Anordnung nach einem der Ansprüche 1 bis 3,
**dadurch gekennzeichnet, dass** die Dünnschicht als Inhibitor für den enzymatischen Abbau einen photoaktivierbaren kettenverlängernden oder vemetzenden Radikalbildner enthält.

9. Anordnung nach Anspruch 8,
**dadurch gekennzeichnet, dass** der Radikalbildner eine erhöhte spektrale Empfindlichkeit in einem Wellenlängenbereich von 240 nm bis 550 nm aufweist.

10. Anordnung nach Anspruch 8 oder 9,
**dadurch gekennzeichnet, dass** die Dünnschicht einen Radikalbildner auf Diazidostilbenbasis enthält.

11. Anordnung nach Anspruch 10,
**dadurch gekennzeichnet, dass** der Diazostilben-Radikalbildner ein Natriumsalz der 4,4-Diazidostilben-2,2-sulfonsäure ist.

12. Verwendung einer Anordnung, umfassend ein Trägersubstrat, auf dem eine Dünnschicht aus einem enzymatisch abbaubaren Biopolymer angebracht ist, die eine Schichtdicke von 30 nm bis 3 µm aufweist und partiell, ganzflächig oder volumenmäßig durchsetzt mit einem Inhibitor für den enzymatischen Abbau versehen ist, zur Herstellung einer mikrostruckturierter Dünnschicht mit darauf immobilisierten Proteinen und/oder Oligonukleotiden auf den Trägersubstrat.

13. Verwendung nach Anspruch 12,
**dadurch gekennzeichnet, dass** die Dünnschicht aus einem enzymatisch abbaubaren Biopolymer, ausgewählt aus der Gruppe bestehend aus Gelatine, Agarose, Dextrose und/oder einem Lipid, gebildet ist.

14. Verwendung nach Anspruch 12 oder 13,
**dadurch gekennzeichnet, dass** die enzymatisch abbaubare Dünnschicht mit lichtempfindlichen Zusätzen als Inhibitoren für den enzymatischen Abbau versehen ist.

15. Verwendung nach einem der Ansprüche 12 bis 14,
**dadurch gekennzeichnet, dass** der Inhibitor eine Verbindung ist, die die Funktion eines das Biopolymer abbauenden Enzyms hemmt.

16. Verwendung nach Anspruch 14 oder 15,
**dadurch gekennzeichnet, dass** der lichtempfindliche Zusatz Diazonaphtochinon ist.

17. Verwendung nach einem der Ansprüche 12 bis 16,
**dadurch gekennzeichnet, dass** die Dünnschicht durch Zusatz thermisch aktivierbarer Reagenzien vernetzt, insbesondere quervernetzt ist.

18. Verwendung nach Anspruch 17,
**dadurch gekennzeichnet, dass** das thermisch aktivierbare Reagenz Glutardialdehyd oder Formaldehyd ist.

19. Verwendung nach einem der Ansprüche 12 bis 14,
**dadurch gekennzeichnet, dass** die Dünnschicht als Inhibitor für den enzymatischen Abbau einen photoaktivierbaren kettenverlängemden oder vemetzenden Radikalbildner enthält.

20. Verwendung nach Anspruch 19,
**dadurch gekennzeichnet, dass** der Radikalbildner eine erhöhte spektrale Empfindlichkeit in einem Wellenlängenbereich von 240 nm bis 550 nm aufweist.

21. Verwendung nach Anspruch 19 oder 20,
**dadurch gekennzeichnet, dass** die Dünnschicht einen Radikalbildner auf Diazidostilbenbasis enthält.

22. Verwendung nach Anspruch 21,
**dadurch gekennzeichnet, dass** der Diazostilben-Radikalbildner ein Natriumsalz der 4,4-Diazidostilben-2,2-sulfonsäure ist.

23. Verfahren zur Herstellung eines Trägersubstrats mit einer mikrostrukturierten Dünnschicht mit vordefinierten Bindungsstellen für Proteine und/oder Oligonukleotide, umfassend die folgenden Schritte:
a) Aufbringen einer Dünnschicht aus einem enzymatisch abbaubaren Biopolymer mit einer Schichtdicke im Bereich von 30 nm bis 3 µm auf ein Trägersubstrat, wobei die Dünnschicht partiell, ganzflächig oder volumenmäßig durchsetzt mit einem Inhibitorfür den enzymatischen Abbau versehen ist und wobei der Inhibitor lichtempfindlich ist oder an einen lichtempfindlichen Zusatz gekoppelt ist;
b) Maskierung der Dünnschicht entsprechend der gewünschten Mikrostruktur der Dünnschicht;
c) Belichtung der maskierten Dünnschicht;
d) Behandlung der belichteten Dünnschicht mit einer Enzymlösung, wobei das Enzym zum Abbau des Biopolymers der Dünnschicht geeignet ist; und
e) Ankoppeln von Proteinen und/oder Oligonukleotiden an das Biopolymer

24. Verfahren nach Anspruch 23,
**dadurch gekennzeichnet, dass** als Trägersubstrat Metall, Polymer, Silizium, beschichtetes Silizium oder Glas verwendet wird.

25. Verfahren nach Anspruch 23 oder 24,
**dadurch gekennzeichnet, dass** der Feststoffgehalt des Biopolymers in der in Schritt a) des Verfahrens aufgebrachten Biopolymer-Lösung 1 bis 30% beträgt.

26. Verfahren nach einem der Ansprüche 23 bis 25,
**dadurch gekennzeichnet, dass** in Schritt a) des Verfahrens Biopolymerschichten aus einer wässrigen Lösung eines Radikalbildners und eines festen, darin zu lösenden Biopolymers in einem Mengenverhältnis von 10:1 bis 100:1, je nach gewünschtem Vernetzungsgrad, gebildet werden.

27. Verfahren nach einem der Ansprüche 23 bis 26,
**dadurch gekennzeichnet, dass** die in Schritt a) des Verfahrens aufgebrachte Lösung ein thermisch aktivierbares Reagenz mit einem Anteil von 1-5% enthält.

28. Anordnung nach einem der Ansprüche 1 bis 11, wobei auf dem Trägersubstrat die Dünnschicht aus einem enzymatisch abbaubaren Biopolymer in Form einer mikrostrukturierten Matrix angebracht ist und, wobei die Matrix lokale Bindungsstellen definiert, an die Proteine und/oder Oligonukleotide angekoppelt sind.

29. Verwendung einer Anordnung nach einem der Ansprüche 1 bis 11 bzw. nach Anspruch 28 zur Immobilisierung von Substanzbibliotheken.

30. Verwendung einer Anordnung nach einem der Ansprüche 1 bis 11 bzw. nach Anspruch 28 in Screening-Tests.

## Claims

1. Arrangement comprising a carrier substrate, on which a thin layer made of an enzymatically degradable biopolymer is applied, the thin layer having a layer thickness of 30 nm to 3 µm and being provided with an inhibitor for enzymatic degradation, partially, over the total surface or volumetrically intermingled, and wherein proteins and/or oligonucleotides are coupled to the biopolymer.

2. Arrangement according to claim 1,
**characterized in that** the thin layer is composed of an enzymatically degradable biopolymer, selected from the group consisting of gelatine, agarose, dextrose and/or a lipid.

3. Arrangement according to claim 1 or 2,
**characterized in that** the enzymatically degradable thin layer is provided with light-sensitive additives as inhibitors for the enzymatic degradation.

4. Arrangement according to any of claims 1 to 3,
**characterized in that** the inhibitor is a compound which inhibits the function of an enzyme degrading the biopolymer.

5. Arrangement according to claim 3 or 4,
**characterized in that** the light-sensitive additive is diazonaphtoquinone.

6. Arrangement according to any of the preceding claims,
**characterized in that** the thin layer is linked, especially cross-linked, by adding thermally activatable reagents.

7. Arrangement according to claim 6,
**characterized in that** the thermally activatable reagent is glutardialdehyde or formaldehyde.

8. Arrangement according to any of claims 1 to 3,
**characterized in that** the thin layer contains a photoactivatable chain-elongating or cross-linking radical former as inhibitor for the enzymatic degradation.

9. Arrangement according to claim 8,
**characterized in that** the radical former exhibits increased spectral sensitivity in a wavelength range of 240 nm to 550 nm.

10. Arrangement according to claim 8 or 9,
**characterized in that** the thin layer contains a radical former on the basis of diazidostilbene.

11. Arrangement according to claim 10,
**characterized in that** the diazidostilbene radical former is a sodium salt of 4,4-diazidostilbene-2,2-sulfonic acid.

12. Use of an arrangement, comprising a carrier substrate, on which a thin layer made of an enzymatically degradable biopolymer is applied, the thin layer having a layer thickness of 30 nm to 3 µm and being provided with an inhibitor for enzymatic degradation, partially, over the total surface or volumetrically intermingled, for the manufacture of a microstructured thin layer with proteins and/or oligonucleotides immobilized thereon on the carrier substrate.

13. Use according to claim 12,
**characterized in that** the thin layer is composed of an enzymatically degradable biopolymer, selected from the group consisting of gelatine, agarose, dextrose and/or a lipid.

14. Use according to claim 12 or 13,
**characterized in that** the enzymatically degradable thin layer is provided with light-sensitive additives as inhibitors for the enzymatic degradation.

15. Use according to any of claims 12 to 14,
**characterized in that** the inhibitor is a compound which inhibits the function of an enzyme degrading the biopolymer.

16. Use according to claim 14 or 15,
**characterized in that** the light-sensitive additive is diazonaphtoquinone.

17. Use according to any of claims 12 to 16,
**characterized in that** the thin layer is linked, especially cross-linked, by adding thermally activatable reagents.

18. Use according to claim 17,
**characterized in that** the thermally activatable reagent is glutardialdehyde or formaldehyde.

19. Use according to any of claims 12 to 14,
**characterized in that** the thin layer contains a photoactivatable chain-elongating or cross-linking radical former as inhibitor for the enzymatic degradation.

20. Use according to claim 19,
**characterized in that** the radical former exhibits increased spectral sensitivity in a wavelength range of 240 nm to 550 nm.

21. Use according to claim 19 or 20,
**characterized in that** the thin layer contains a radical former on the basis of diazidostilbene.

22. Use according to claim 21,
**characterized in that** the diazidostilbene radical former is a sodium salt of 4,4-diazidostilbene-2,2-sulfonic acid.

23. Method for the manufacturing of a carrier substrate having a microstructured thin layer with predefined binding sites for proteins and/or oligonucleotides, comprising the following steps:
a) applying a thin layer made of an enzymatically degradable biopolymer with a layer thickness in the range of 30 nm to 3 µm onto a carrier substrate, wherein the thin layer is provided, partially, over the total surface or volumetrically intermingled, with an inhibitor for enzymatic degradation and wherein the inhibitor is light-sensitive or coupled to a light-sensitive additive;
b) masking the thin layer according to the desired microstructure of the thin layer;
c) exposing the masked thin layer to light;
d) treating the light-exposed thin layer with an enzyme solution, the enzyme being capable of degrading the biopolymer of the thin layer; and
e) coupling proteins and/or oligonucleotides to the biopolymer

24. Method according to claim 23,
**characterized in that** metal, polymer, silicon, coated silicon or glass are used as carrier substrate.

25. Method according to claim 23 or 24,
**characterized in that** the solids content of the biopolymer in the biopolymer solution applied in step a) of the method is 1 to 30 %.

26. Method according to any of claims 23 to 25,
**characterized in that** biopolymer layers are generated in step a) of the method from an aqueous solution of a radical former and of a solid biopolymer to be dissolved therein in a quantitative ratio of 10:1 1 to 100:1 1 depending on degree of cross-linking desired.

27. Method according to any of claims 23 to 26,
**characterized in that** the solution applied in step a) of the method contains 1-5% of a thermally activatable reagent

28. Arrangement according to any of claims 1 to 11, wherein the thin layer made of an enzymatically degradable biopolymer is applied on the carrier substrate in the form of a microstructured matrix, the matrix defining local binding sites to which proteins and/or oligonucleotides are coupled.

29. Use of an arrangement according to any of claims 1 to 11 or according to claim 28 for the immobilization of substance libraries.

30. Use of an arrangement according to any of claims 1 to 11 or according to claim 28 in screening tests.

## Revendications

1. Dispositif comportant un substrat porteur sur lequel est déposée une couche mince d'un biopolymère dégradable par enzymes qui présente une épaisseur de couche de 30 nm à 3 µm et qui est pourvue, partiellement, sur toute la surface ou traversée en volume, d'un inhibiteur de la dégradation enzymatique, des protéines et/ou des oligonucléotides étant couplés au biopolymère.

2. Dispositif selon la revendication 1,
**caractérisé en ce que** la couche mince est formée d'un biopolymère dégradable par enzymes, choisie dans le groupe se composant de la gélatine, de l'agarose, du dextrose et/ou d'un lipide.

3. Dispositif selon la revendication 1 ou 2,
**caractérisé en ce que** la couche mince dégradable par enzymes est pourvue d'additifs photosensibles en tant qu'inhibiteurs de la dégradation enzymatique.

4. Dispositif selon l'une quelconque des revendications 1 à 3,
**caractérisé en ce que** l'inhibiteur est une liaison qui inhibe la fonction d'une enzyme dégradant le biopolymère.

5. Dispositif selon la revendication 3 ou 4,
**caractérisé en ce que** l'additif photosensible est de la diazonaphtoquinone.

6. Dispositif selon l'une quelconque des revendications précédentes,
**caractérisé en ce que** la couche mince est réticulée par ajout de réactifs thermiquement activables, en particulier réticulée transversalement.

7. Dispositif selon la revendication 6,
**caractérisé en ce que** le réactif thermiquement activable est du dialdéhyde glutarique ou de l'aldéhyde formique.

8. Dispositif selon l'une quelconque des revendications 1 à 3,
**caractérisé en ce que** la couche mince en tant qu'inhibiteur de la dégradation enzymatique contient un formateur photoactivable de radical, réticulant ou allongeant la chaîne.

9. Dispositif selon la revendication 8,
**caractérisé en ce que** le formateur de radical présente une sensibilité spectrale plus forte dans une plage de longueurs d'onde de 240 nm à 550 nm.

10. Dispositif selon la revendication 8 ou 9,
**caractérisé en ce que** la couche mince contient un formateur de radical à base de diazidostilbene.

11. Dispositif selon la revendication 10,
**caractérisé en ce que** le formateur de radical diazidostilbene est un sel de sodium de 4,4-diazidostilbene-2,2-acide sulfonique.

12. Utilisation d'un dispositif comprenant un substrat porteur sur lequel est déposée une couche mince d'un biopolymère dégradable par enzymes qui présente une épaisseur de couche de 30 nm à 3 µm et qui est pourvue partiellement, sur toute la surface ou traversée en volume, par un inhibiteur de la dégradation enzymatique, pour fabriquer une couche mince microstructurée, comportant sur elle des protéines immobilisées et/ou des oligonucléotides sur le substrat porteur.

13. Utilisation selon la revendication 12,
**caractérisée en ce que** la couche mince est formée d'un biopolymère dégradable par enzymes, choisie dans le groupe se composant de la gélatine, de l'agarose, du dextrose et/ou d'un lipide.

14. Utilisation selon la revendication 12 ou 13,
**caractérisée en ce que** la couche mince dégradable par enzymes est pourvue d'additifs photosensibles en tant qu'inhibiteurs de la dégradation enzymatique.

15. Utilisation selon l'une quelconque des revendications 12 à 14,
**caractérisée en ce que** l'inhibiteur est une liaison qui inhibe la fonction d'une enzyme dégradant le biopolymère.

16. Utilisation selon la revendication 14 ou 15,
**caractérisée en ce que** l'additif photosensible est de la diazonaphtoquinone.

17. Utilisation selon l'une quelconque des revendications 12 à 16,
**caractérisée en ce que** la couche mince est réticulée par ajout de réactifs thermiquement activables, en particulier réticulée transversalement.

18. Utilisation selon la revendication 17,
**caractérisée en ce que** le réactif thermiquement activable est du dialdéhyde glutarique ou de l'aldéhyde formique.

19. Utilisation selon l'une quelconque des revendications 12 à 14,
**caractérisée en ce que** la couche mince en tant qu'inhibiteur de la dégradation enzymatique contient un formateur photoactivable de radical, réticulant ou allongeant la chaîne.

20. Utilisation selon la revendication 19,
**caractérisée en ce que** le formateur de radical présente une sensibilité spectrale plus forte dans une plage de longueurs d'onde de 240 nm à 550 nm.

21. Utilisation selon la revendication 19 ou 20,
**caractérisée en ce que** la couche mince contient un formateur de radical à base de diazidostilbene.

22. Utilisation selon la revendication 21,
**caractérisée en ce que** le formateur de radical diazidostilbene est un sel de sodium de 4,4-Diazidostilbene-2,2-acide sulfonique.

23. Procédé de fabrication d'un substrat porteur comportant une couche mince microstructurée avec des emplacements de liaison prédéfinis pour des protéines et/ou des oligonucléotides, comportant les étapes suivantes :
a) dépôt d'un substrat porteur d'une couche mince d'un biopolymère dégradable par enzymes d'une épaisseur de couche dans la plage des 30 nm à 3 µm, la couche mince étant pourvue, partiellement, sur toute la surface ou traversée en volume, d'un inhibiteur de la dégradation enzymatique, et l'inhibiteur étant photosensible ou couplé à un additif photosensible ;
b) masquage de la couche mince en fonction de la microstructure souhaitée de la couche mince ;
c) illumination de la couche mince masquée ;
d) traitement de la couche mince avec une solution d'enzyme, l'enzyme étant adaptée à la dégradation du biopolymère de la couche mince ; et
e) couplage de protéines et/ou d'oligonucléotides au biopolymère.

24. Procédé selon la revendication 23,
**caractérisé en ce que**, en tant que substrat porteur, du métal, du polymère, du silicium, du silicium ou du verre revêtu, est utilisé.

25. Procédé selon la revendication 23 ou 24,
**caractérisé en ce que** la teneur en matériau solide du biopolymère de la solution de biopolymère déposée à l'étape a) du procédé est de 1 à 30 %.

26. Procédé selon l'une quelconque des revendications 23 à 25,
**caractérisé en ce que**, à l'étape a) du procédé, sont formées des couches de biopolymère d'une solution aqueuse d'un formateur de radical et d'un biopolymère solide à y dissoudre dans un rapport de quantités de 10 : 1 à 100 : 1 selon le degré de réticulation souhaité.

27. Procédé selon l'une quelconque des revendications 23 à 26,
**caractérisé en ce que** la solution déposée à l'étape a) du procédé contient dans une proportion de 1 à 5 % un réactif thermiquement activable.

28. Dispositif selon l'une quelconque des revendications 1 à 11, la couche mince d'un biopolymère dégradable par enzyme étant déposée sur le substrat porteur sous la forme d'une matrice microstructurée, et la matrice définissant des emplacements locaux de liaison auxquels des protéines et/ou des oligonucléotides sont couplés.

29. Utilisation d'un dispositif selon l'une quelconque des revendications 1 à 11 ou selon la revendication 28 pour immobiliser des bibliothèques de substances.

30. Utilisation d'un dispositif selon l'une quelconque des revendications 1 à 11 ou selon la revendication 28 dans des tests de sélection.
